# FASCICULE DE BREVET EUROPEEN

(11) **EP 4 402 468 B1**
(45) Date de publication et mention de la délivrance du brevet: **08.04.2026**
(21) Numéro de dépôt: 22769398.3
(22) Date de dépôt: 13.09.2022
(51) Int. Cl.: G01N 33/18, E04H 4/10, E04H 4/08, E04H 4/14

(54) **DISPOSITIF D'ANALYSE D'UN MILIEU LIQUIDE, EAU D'UNE PISCINE NOTAMMENT**
VORRICHTUNG ZUR ANALYSE EINES FLÜSSIGEN MEDIUMS, INSBESONDER WASSER EINES SCHWIMMBECKENS
DEVICE FOR ANALYSING A LIQUID MEDIUM, IN PARTICULAR WATER OF A SWIMMING POOL

(30) Priorité: 17.09.2021 FR 2109763
(43) Date de publication de la demande: 24.07.2024
(73) Titulaire: Bleu Electrique, 13016 Marseille (FR)
(72) Inventeur: BARET, Emmanuel, 13016 MARSEILLE (FR)
(74) Mandataire: Hautier IP
(86) Numéro de dépôt international: PCT/IB2022/058616
(87) Numéro de publication internationale: WO 2023/042071

(56) Documents cités:
- FR-A1- 3 064 360
- US-A1- 2014 007 338
- US-A1- 2019 108 745

## Description

Le domaine de la présente invention est celui des dispositifs d'analyse d'un milieu liquide, eau d'une piscine notamment. Elle a pour objet un tel dispositif d'analyse. Elle a aussi pour objet un ensemble comprenant un tel dispositif d'analyse et un volet de protection destiné à flotter à la surface de l'eau de la piscine. Elle a aussi pour objet une piscine pourvue d'un tel ensemble.

Le document FR3064360 décrit un dispositif d'analyse d'un milieu liquide comprenant un corps flottant à la surface d'un milieu liquide, tel que l'eau d'une piscine. Le corps comprend une partie étanche comportant une unité de commande du dispositif d'analyse relié à un module de communication, une source lumineuse comprenant au moins deux couleurs, un accéléromètre et une base de données. La partie étanche est émergée et surplombe la surface de l'eau de la piscine. La partie étanche est globalement sphérique et comprend une cellule photovoltaïque permettant de recharger une batterie que comprend également le dispositif d'analyse. Le corps comprend aussi une partie immergée comprenant des capteurs pour mesurer des caractéristiques de qualité du milieu liquide, telles que le pH, la conductivité électrique, la température, le potentiel d'électro-réduction, les capteurs étant reliés à l'unité de commande. La partie émergée est globalement cylindrique et comporte une zone évasée qui rejoint la partie étanche sphérique.

Un tel dispositif d'analyse est notamment destiné à analyser l'eau d'une piscine dotée d'un volet de protection. Ce volet de protection est prévu pour empêcher une chute d'une personne, notamment d'un enfant, à l'intérieur de la piscine afin d'éviter une noyade de ce dernier. Ce volet de protection est également prévu pour empêcher des salissures, feuilles d'arbre ou analogue, de tomber dans l'eau de la piscine et de la souiller. Ce volet de protection permet aussi une isolation thermique de l'eau de la piscine, par exemple lors d'une période de non-utilisation de la piscine, ce qui vise à minimiser un refroidissement de l'eau de la piscine.

A cet effet, le volet de protection est mobile entre une position de couverture dans laquelle le volet de protection recouvre l'eau de la piscine en flottant à la surface de cette dernière et une position d'escamotage dans laquelle le volet de protection est enroulé autour d'un axe de rangement situé à proximité d'un rebord de la piscine. Le volet de protection est couramment manœuvré entre la position d'escamotage et la position de couverture jusqu'à ce qu'une extrémité du volet de protection vienne en contact avec le rebord de la piscine.

Un problème général dans le domaine réside dans une impossibilité d'utiliser un tel dispositif d'analyse lorsque le volet de protection est manœuvré depuis la position d'escamotage vers la position de couverture. En effet, la partie étanche, émergée, forme un obstacle au déplacement du volet de protection jusqu'à être coincée entre l'extrémité du volet de protection et le bord de la piscine. Il en résulte une possibilité de déformation, voire de détérioration, du volet de protection et/ou du dispositif d'analyse.

Un autre problème général dans le domaine réside dans une impossibilité d'utiliser un tel dispositif d'analyse lorsque le volet de protection est placé en position de couverture. En effet, en position de couverture du volet de protection, le dispositif d'analyse risque d'être totalement immergé. Il en résulte une sollicitation préjudiciable d'une étanchéité du dispositif d'analyse qui est pourtant souhaitée parfaite au niveau de la partie étanche. Or, un séjour prolongé dans l'eau peut provoquer une altération de l'étanchéité de la partie étanche sur le long terme. De plus, lorsque le dispositif d'analyse est immergé et recouvert par le volet de protection, un fonctionnement du module de communication est susceptible d'être altéré, voire arrêté, le volet de protection et/ou l'eau formant un écran néfaste au fonctionnement du module de communication. De plus encore, lorsque le dispositif d'analyse est immergé et recouvert par le volet de protection, la cellule photovoltaïque ne capte plus les rayons lumineux et devient de ce fait inapte à recharger la batterie, ce qui diminue fortement une autonomie du dispositif d'analyse.

Une possibilité est d'ôter le dispositif d'analyse de la piscine lors de la manœuvre du volet de protection sur la piscine. Or, il est souhaitable de disposer en continu des caractéristiques de qualité du milieu liquide, pour éviter toute dérive de ces derniers et adapter rapidement un traitement du milieu liquide, si besoin. Cette possibilité s'avère donc contraignante et finalement insatisfaisante.

Une autre possibilité est de laisser le volet de protection recouvrir le dispositif d'analyse et d'immerger entièrement ce dernier dans l'eau de la piscine. Comme évoqué, cette possibilité entraine des dysfonctionnements du module de communication, de la cellule photovoltaïque et altère l'étanchéité du dispositif d'analyse. Cette possibilité s'avère donc insatisfaisante
Une autre possibilité est de ne pas déployer entièrement le volet de protection lorsque le dispositif d'analyse est en place ce qui s'avère dangereux, un enfant étant susceptible de tomber dans la piscine dans un interstice formé entre l'extrémité du volet de protection et le rebord de la piscine, le volet de protection pouvant alors empêcher l'enfant de refaire surface et de s'extraire de la piscine. Cette possibilité est donc à proscrire.

La présente invention s'inscrit dans ce contexte et propose un dispositif d'analyse d'un milieu liquide, eau d'une piscine notamment. Le dispositif d'analyse comprend une enveloppe logeant des moyens d'analyse du milieu liquide pour mesurer des paramètres physico-chimiques du milieu liquide, des moyens de contrôle pour gérer les paramètres mesurés, des moyens de communication aptes à transmettre lesdits paramètres à une unité centrale, au moins un panneau photovoltaïque en relation avec des moyens de stockage d'énergie électrique pour alimenter en énergie électrique les moyens d'analyse, les moyens de contrôle et les moyens de communication.

Selon la présente invention, l'enveloppe comprend un organe de préhension qui est agencé en U et qui délimite un espace apte à recevoir une extrémité d'un volet de protection.

Le dispositif d'analyse comprend avantageusement l'une quelconque au moins des caractéristiques techniques suivantes, prises seules ou en combinaison :
- l'organe de préhension comprend une première paroi constitutive d'une première partie étanche, une deuxième paroi constitutive d'une deuxième partie immergée comprenant une base pourvue d'une pluralité de lumières et logeant les moyens d'analyse, et une troisième paroi pourvue d'une butée de volet agencée pour recevoir l'extrémité du volet de protection,
- l'organe de préhension est pourvu d'un moyen de détection d'une présence du volet de protection à l'intérieur de l'espace, le moyen de détection comprenant un premier organe de détection équipant la première paroi et un deuxième organe de détection équipant la deuxième paroi,
- la première paroi borde une première chambre qui loge le panneau photovoltaïque et qui est surplombée par un capot réalisé en une matière translucide et phosphorescente,
- la deuxième paroi borde une deuxième chambre qui loge les moyens de contrôle et les moyens de stockage d'énergie électrique, la deuxième chambre étant partiellement délimitée par une cloison d'étanchéité qui est constitutive d'un couvercle d'étanchéité et qui obture une ouverture de la base,
- le couvercle d'étanchéité comprend une collerette périphérique constitutive de l'enveloppe, la collerette périphérique étant interposée entre un chapeau du dispositif d'analyse et la base,
- la troisième paroi borde une troisième chambre qui loge les moyens de communication,
- la troisième chambre est bordée par une quatrième paroi pourvue d'un premier organe d'ancrage du dispositif d'analyse.

La présente invention a aussi pour objet un ensemble comprenant un tel dispositif d'analyse et le volet de protection.

La présente invention a aussi pour objet une piscine équipée d'un tel ensemble.

D'autres caractéristiques et avantages de l'invention apparaîtront encore au travers de la description qui suit d'une part, et de plusieurs exemples de réalisation donnés à titre indicatif et non limitatif en référence aux dessins schématiques annexés d'autre part, sur lesquels :
[Fig.1] est une illustration schématique de côté d'un dispositif d'analyse d'un milieu liquide de la présente invention, en une première position d'utilisation du dispositif d'analyse, dans laquelle le dispositif d'analyse est ancré contre un rebord d'une piscine.
[Fig.2] est une illustration schématique de côté du dispositif d'analyse illustré sur la [Fig.1], et représenté en une deuxième position d'utilisation du dispositif d'analyse, dans laquelle le dispositif d'analyse est libre flottant dans le milieu liquide.
[Fig.3] est une illustration schématique de côté d'un ensemble comprenant le dispositif d'analyse illustré sur les figures 1 et 2, et un volet de protection en cours de déplacement entre une position d'escamotage et une position de couverture dans laquelle le volet de protection recouvre le milieu liquide.
[Fig.4] est une illustration schématique de dessus de l'ensemble représenté sur la [Fig.3], le volet de protection étant illustré en position de couverture.
[Fig.5] est une illustration schématique de dessus de l'ensemble représenté sur les figures 3 et 4, le volet de protection étant illustré en position de d'escamotage.
[Fig.6] est une illustration schématique de côté de l'ensemble illustré sur les figures 3 à 5, le volet de protection étant illustré en position de couverture.
[Fig.7] est une illustration schématique de côté du dispositif d'analyse illustré sur les figures 1 et 2.
[Fig.8] est une illustration schématique de côté de l'ensemble illustré sur les figures 3 à 6.
[Fig.9] est une illustration schématique en coupe du dispositif d'analyse illustré sur les figures 1, 2 et 7.
[Fig.10] est une illustration schématique en perspective d'une base constitutive du dispositif d'analyse illustré sur les figures 1, 2, 7 et 9.
[Fig.11] est une illustration schématique en perspective de dessus d'un couvercle d'étanchéité constitutif du dispositif d'analyse illustré sur les figures 1, 2, 7 et 9.
[Fig.12] est une illustration schématique en perspective de dessous du couvercle d'étanchéité illustré sur la [Fig.11].
[Fig.13] est une illustration schématique de côté d'un chapeau constitutif du dispositif d'analyse illustré sur les figures 1, 2, 7 et 9.

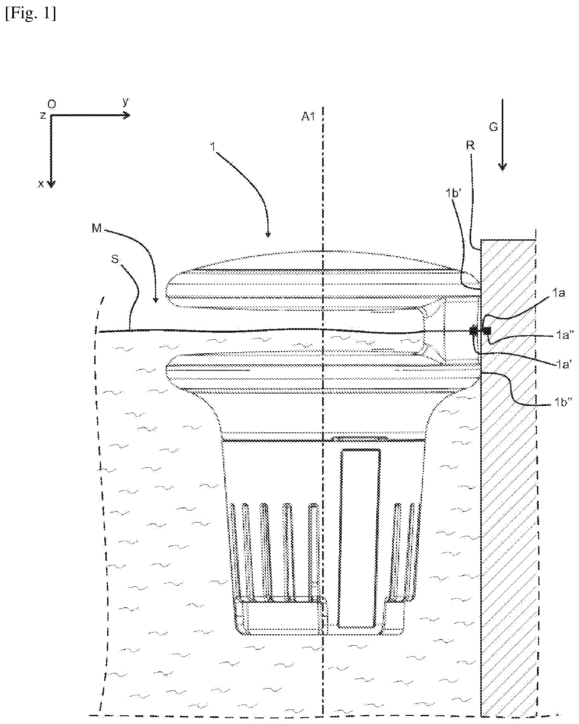

Sur les figures 1 à 9, un dispositif d'analyse 1 d'un milieu liquide M de la présente invention est représenté à l'intérieur d'un repère orthonormé Oxyz. Le repère orthonormé Oxyz comprend un axe Ox qui est un axe vertical, parallèle à l'axe de gravité terrestre G. Le repère orthonormé Oxyz comprend aussi un plan Oyz qui est un plan horizontal, orthogonal à l'axe de gravité terrestre G.

Sur les figures 1 à 3, le dispositif d'analyse 1 est destiné à analyser le milieu liquide M, telle que l'eau d'une piscine, d'un jacuzzi ou analogue. Le dispositif d'analyse 1 s'étend principalement selon un axe d'allongement A1, qui est parallèle à l'axe Ox, en positions d'utilisation du dispositif d'analyse 1 à l'intérieur du milieu liquide M, telles que représentées sur les figures 1 à 6. On note qu'en position d'utilisation du dispositif d'analyse 1 à l'intérieur du milieu liquide M, l'axe d'allongement A1 est sensiblement orthogonal à une surface S du milieu liquide M, ci-après dénommé l'eau.

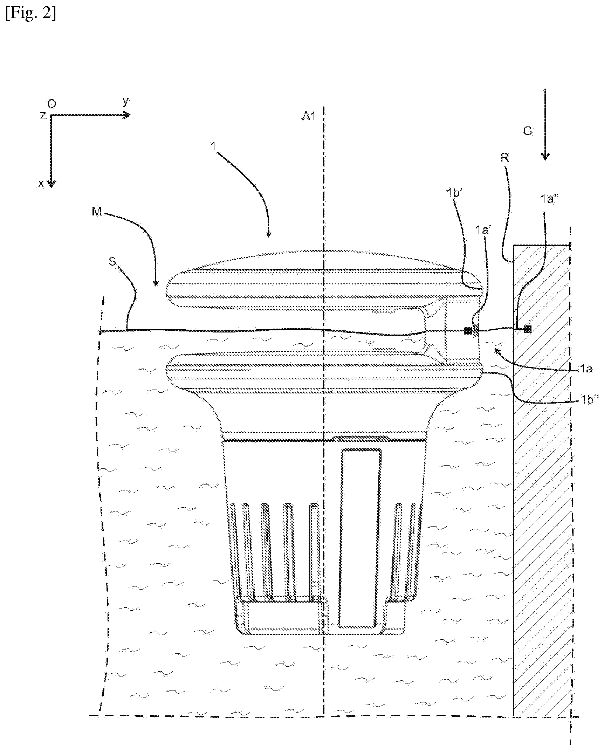

Sur la [Fig.1], le dispositif d'analyse 1 est représentée en une première position d'utilisation dans laquelle le dispositif d'analyse 1 est ancré à un premier rebord R de la piscine par l'intermédiaire d'un moyen d'ancrage 1a. Le moyen d'ancrage 1a est indifféremment un moyen d'ancrage magnétique, mécanique ou analogue. Le moyen d'ancrage 1a est conformé de manière à ce que l'ancrage soit réversible. Autrement dit, le moyen d'ancrage 1a permet soit de maintenir le dispositif d'analyse 1 en la première position d'utilisation contre le premier rebord R de la piscine, tel qu'illustré sur la [Fig.1], soit de laisser librement flotter le dispositif d'analyse 1, tel qu'illustré sur la [Fig.2]. On comprend que le moyen d'ancrage 1a est par exemple constitué d'un premier organe d'ancrage 1a' et d'un deuxième organe d'ancrage 1a", affectés respectivement au dispositif d'analyse 1 et au premier rebord R de la piscine. Les organes d'ancrage 1a', 1a" sont par exemple formés d'un couple d'aimants, d'une boucle et d'un crochet, ou analogue, qui sont ménagés au niveau de la surface S de l'eau.

On note aussi que le dispositif d'analyse 1 comprend au moins une butée de dispositif 1b', 1b", et préférentiellement deux butées de dispositif 1b', 1b", qui sont conformées pour venir au contact du premier rebord R de la piscine, par exemple à partir d'un épaississement de matière du dispositif d'analyse 1.

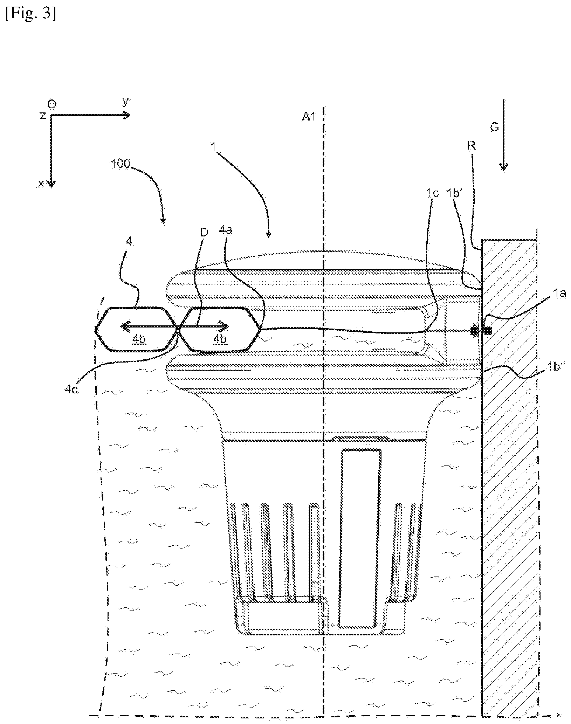

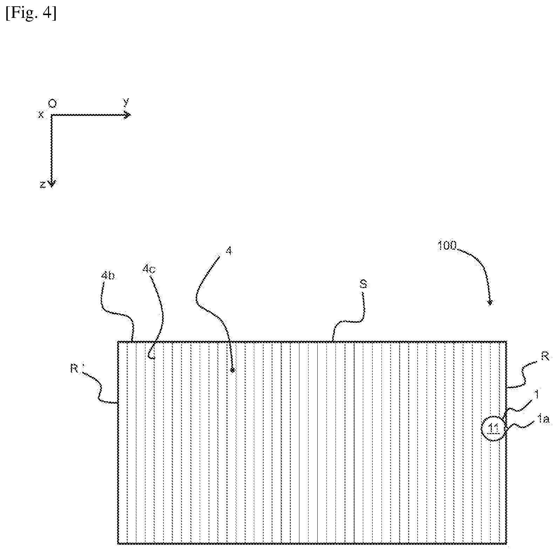

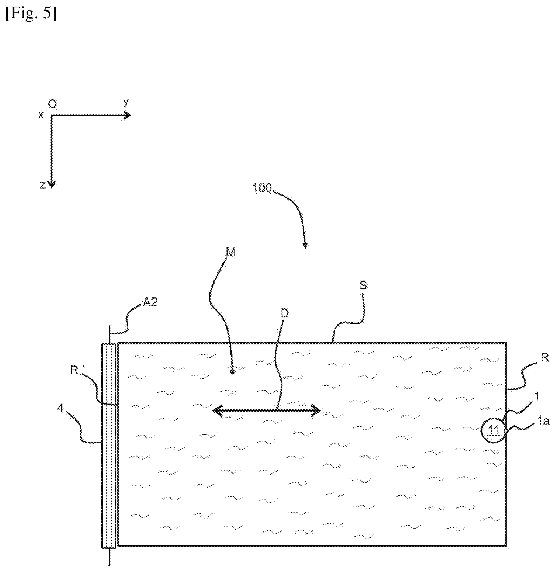

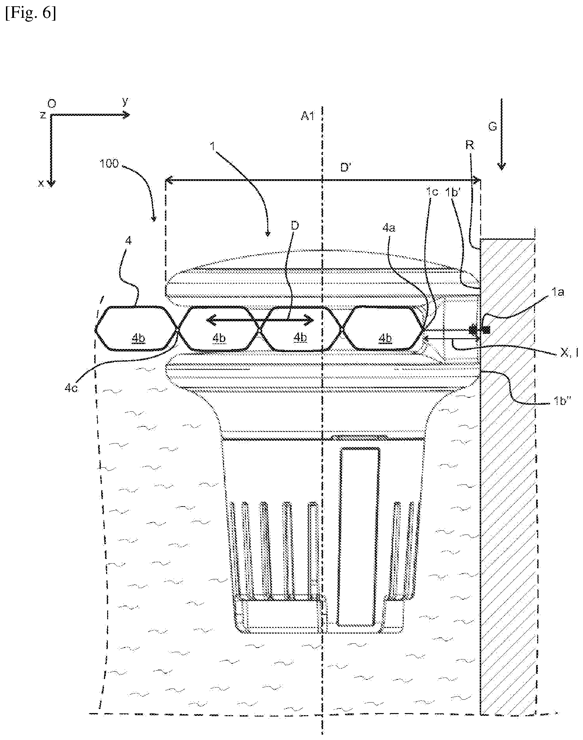

Sur la [Fig.3], est représenté un ensemble 100 comprenant le dispositif d'analyse 1 et un volet de protection 4 de l'eau de la piscine. Dans sa généralité, le volet de protection 4 est mobile entre une position de couverture, illustrée sur la [Fig.4], dans laquelle le volet de protection 4 recouvre l'eau de la piscine en flottant à la surface S de cette dernière et une position d'escamotage, illustrée sur la [Fig.5], dans laquelle, par exemple, le volet de protection 4 est enroulé autour d'un axe de rangement A2 situé à proximité d'un deuxième rebord R' de la piscine, notamment situé à l'opposé du premier rebord R de la piscine équipé du deuxième organe d'ancrage 1a", tel qu'illustré sur la [Fig.5]. De préférence, le volet de protection 4 est formé de lattes 4b qui sont reliées deux à deux par un moyen d'articulation 4c. Autrement dit, le volet de protection 4 est destiné à subir un déplacement D entre la position d'escamotage et la position de couverture jusqu'à ce qu'une extrémité 4a du volet de protection 4 vienne en contact avec une butée de volet 1c du dispositif d'analyse 1, de telle sorte que le dispositif d'analyse 1 est bloqué entre l'extrémité 4a du volet de protection 4 et le rebord R de la piscine, tel qu'illustré sur la [Fig.6].

On note qu'une distance X prise orthogonalement à l'axe d'allongement A1 entre la butée de volet 1c et l'une des butées de dispositif 1b', 1b" est la plus réduite possible, notamment de l'ordre de quelques centimètres, de telle sorte qu'un interstice I pris orthogonalement à l'axe d'allongement A1 entre l'extrémité de volet 4a et le rebord R de la piscine est le plus réduit possible également, l'interstice I et la distance X étant sensiblement équivalents l'un à l'autre. On note que l'interstice I et/ou la distance X sont notamment inférieurs à 25%, voire et préférentiellement à 10%, d'une dimension D' du dispositif d'analyse 1 prise dans un plan sensiblement orthogonal à l'axe d'allongement A1. La dimension D' est par exemple un diamètre du dispositif d'analyse 1 globalement cylindrique dont l'axe d'allongement A1 forme un axe de révolution.

Cette disposition avantageuse est atteinte à partir d'un agencement particulier du dispositif d'analyse 1 décrit ci-après.

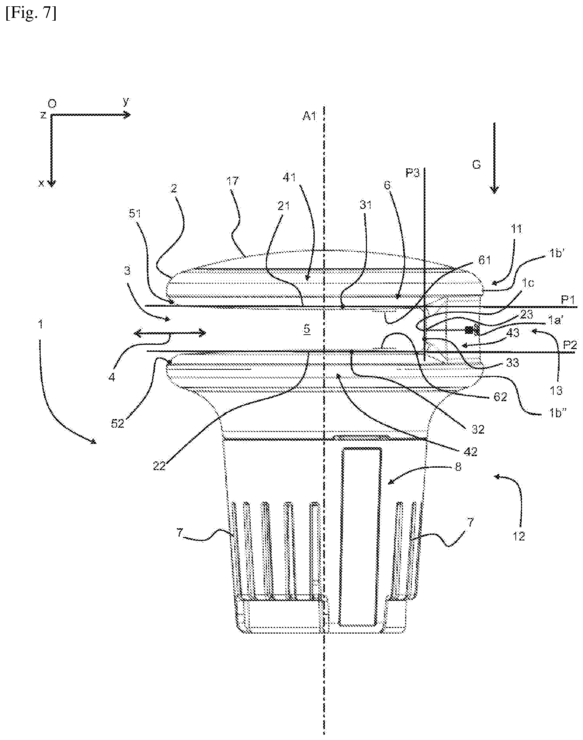

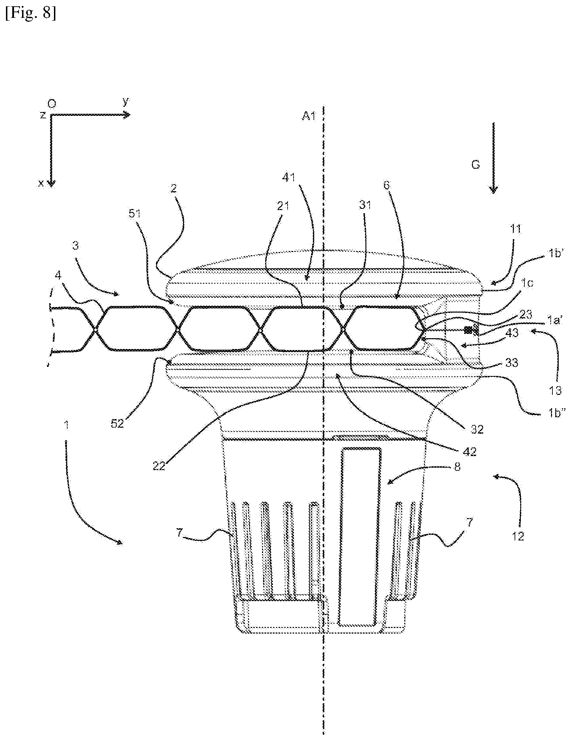

Sur les figures 7 et 8, le dispositif d'analyse 1 comprend une première partie 11, notamment supérieure en position d'utilisation du dispositif d'analyse 1, et une deuxième partie 12, notamment inférieure en position d'utilisation du dispositif d'analyse 1, qui sont reliées l'une à l'autre par une partie intermédiaire 13. On comprend que la partie intermédiaire 13 est interposée entre la première partie 11 et la deuxième partie 12 et qu'en position d'utilisation la première partie 11 surplombe la deuxième partie 12 et la partie intermédiaire 13. Autrement dit, en parcourant l'axe d'allongement A1, on trouve successivement la première partie 11, la partie intermédiaire 13, puis la deuxième partie 12. La première partie 11 est une partie étanche prévue pour émerger au-dessus de la surface S de l'eau tandis que la deuxième partie 12 est destinée à être immergée à l'intérieur de l'eau de la piscine, tel que cela est illustré sur la [Fig.4].

On comprend aussi que la première partie 11 surplombe le volet de protection 4 en position de recouvrement, tel qu'illustré sur la [Fig.4].

Le dispositif d'analyse 1 comprend une enveloppe 2 qui délimite la première partie 11, la deuxième partie 12 et la troisième partie 13. L'enveloppe 2 constitue une surface externe du dispositif d'analyse 1 visible depuis l'extérieur de celui-ci. L'enveloppe 2 comporte avantageusement un organe de préhension 3 qui est apte à enserrer le volet de protection 4 de la piscine, tel que représenté sur les figures 4, 6 et 8.

On comprend que l'organe de de préhension 3 est conformé pour ceindre le volet de protection 4. Autrement dit, l'organe de préhension 3 est agencé de telle sorte que le volet de protection 4 peut s'emboîter à l'intérieur de l'organe de préhension 3, jusqu'à ce que l'extrémité 4a du volet de protection 4 vienne en contact contre la butée de volet 1c du dispositif d'analyse 1. A cet effet, l'organe de préhension 3 est agencé en U dont une première branche 21 est constitutive de la première partie 11, dont une deuxième branche 22 est constitutive de la deuxième partie 12 et dont une base 23 est constitutive de la partie intermédiaire 13. Ainsi, l'organe de préhension 3 comprend une première paroi 31 qui est constitutive de la première branche 21 du U et qui délimite partiellement une première chambre 41 de la première partie 11. L'organe de préhension 3 comprend également une deuxième paroi 32 qui est constitutive de la deuxième branche 22 du U et qui délimite partiellement une deuxième chambre 42 de la deuxième partie 12. L'organe de préhension 3 comprend aussi une troisième paroi 33 qui est constitutive de la base 23 du U et qui délimite une troisième chambre 43 de la partie intermédiaire 13. La troisième paroi 33 comporte avantageusement la butée de volet 1c du dispositif d'analyse 1. La première paroi 21 s'étend principalement selon un premier plan P1 qui est orthogonal à l'axe d'allongement A1, la deuxième paroi 22 s'étend principalement à l'intérieur d'un deuxième plan P2, distinct et parallèle au premier plan P1 tandis que la troisième paroi 23 s'étend sensiblement à l'intérieur d'un troisième plan P3 orthogonal au premier plan P1 et au deuxième plan P2.

La première branche 21 comprend une première extrémité 51, opposée à la base 23, qui est agencée en une rampe de guidage du volet de protection 4 pour faciliter une insertion de ce dernier à l'intérieur de l'organe de préhension 3. De même, la deuxième branche 22 comprend une deuxième extrémité 52, opposée à la base 23, qui est agencée en une rampe de guidage du volet de protection 4 pour faciliter une insertion de ce dernier à l'intérieur de l'organe de préhension 3.

L'organe de préhension 3 est pourvu d'un moyen de détection 6 d'une présence du volet de protection 4 à l'intérieur d'un espace 5 délimité par l'organe de préhension 3 et bordé par la première paroi 31, la deuxième paroi 32 et la troisième paroi 33. Le moyen de détection 6 comprend un premier organe de détection 61 équipant la première paroi 31 et un deuxième organe de détection 62 équipant la deuxième paroi 32. Le moyen de détection 6 est indifféremment un moyen de détection optique, inductif, capacitif ou analogue.

La deuxième partie 12 est pourvue d'une pluralité de lumières 7 ménagées à travers l'enveloppe 2 de telle sorte que l'eau peut pénétrer à l'intérieur de la deuxième partie 12 pour être analysée par des moyens d'analyse 8 que loge la deuxième partie 12. Selon une variante, les lumières 7 sont circulaires. Selon la variante représentée, les lumières 7 sont oblongues.

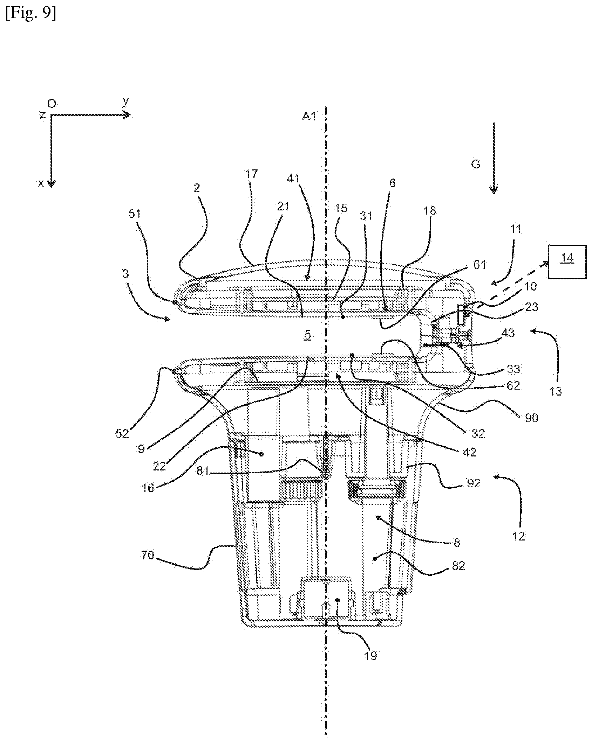

Sur la [Fig.9], les moyens d'analyse 8 comprennent un capteur de température 81 pour mesurer une température de l'eau et des électrodes 82 pour mesurer des paramètres physico-chimiques de l'eau, tels que le pH, le potentiel d'oxydo-réduction, la conductivité électrique, le taux de salinité ou analogue. Les moyens d'analyse 8 sont reliés à des moyens de contrôle 9 qui sont aptes à gérer lesdits paramètres mesurés par le capteur de température 81 et les électrodes 82. Les moyens de contrôle 9 sont reliés à des moyens de communication 10, tels qu'une antenne radiofréquence, une antenne Wifi ou analogue, qui sont aptes à transmettre lesdits paramètres à une unité centrale 14 située à distance du dispositif d'analyse, dans un local technique par exemple. Les moyens de communication 10 sont avantageusement logés à l'intérieur de la troisième chambre 43.

Les moyens d'analyse 8 sont aussi reliés à un panneau photovoltaïque 15, logé dans la première chambre 41, qui est apte à transformer des rayons lumineux en une énergie électrique prévue pour être emmagasinée dans des moyens de stockage d'énergie électrique 16, tels qu'une batterie ou analogue. Le panneau photovoltaïque 15 s'étend par exemple à l'intérieur d'un plan orthogonal à l'axe d'allongement A1.

L'enveloppe 2 comprend un capot 17 qui est réalisé en une matière translucide et phosphorescente pour laisser passer les rayons lumineux vers le panneau photovoltaïque 15. A cet effet, le capot 17 surplombe le panneau photovoltaïque 15 qui surplombe également au moins une diode luminescente 18, et préférentiellement une pluralité de diodes luminescentes RGB ou RGBW qui par la combinaison de leur activation par les moyens de contrôle 9 permet de changer la couleur du capot 17 dans une variation de teintes. Selon les couleurs du capot 17 et/ou leur mode de clignotement, un utilisateur est informé d'un état de fonctionnement du dispositif d'analyse 1, voire du résultat des paramètres mesurés par le capteur de température 81 et les électrodes 82, et notamment d'un franchissement d'un paramètre-seuil pour l'un quelconque de ces paramètres. Le capot 17 comporte des pigments photoluminescents colorés qui accumulent de l'énergie lumineuse durant la journée et la restitue la nuit permettant ainsi au dispositif d'analyse 1 d'être éclairé sans consommer d'énergie électrique.

La deuxième partie 12 loge un lest 19 pour placer le dispositif d'analyse 1 en position d'utilisation, telle que celle définie ci-dessus.

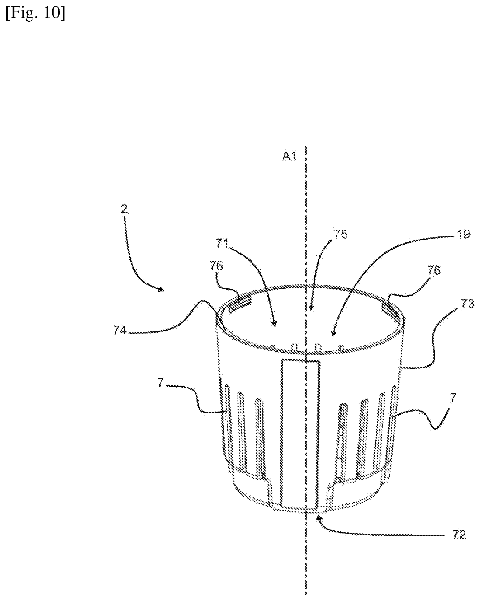

Sur la [Fig.10], l'enveloppe 2 comprend une base 70 agencée en une cuvette qui délimite une enceinte 71 conformée pour loger le lest 19 et pourvue des lumières 7. La base 70 comporte un fond 72 et une paroi cylindrique 73, ou sensiblement cylindrique, qui s'étend entre le fond 72 et un rebord circulaire 74, le rebord circulaire 74 délimitant une ouverture 75. Le fond 72 est équipé du lest 19 tandis que la paroi cylindrique 73 est pourvue des lumières 7. Le fond 72 s'étend dans un plan orthogonal à l'axe d'allongement A1, la paroi cylindrique 73 comporte un axe de symétrie confondu avec l'axe d'allongement A1 et le rebord circulaire 74 s'étend dans un plan orthogonal à l'axe d'allongement A1. La base 70 loge avantageusement les moyens d'analyse 8.

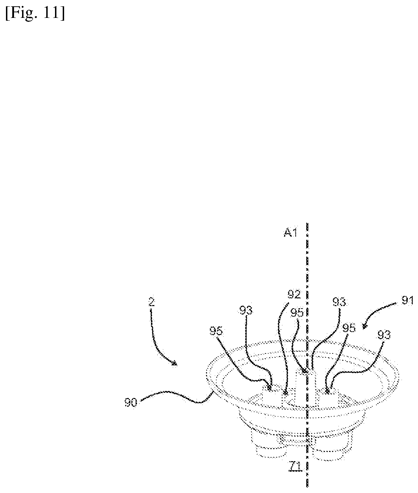

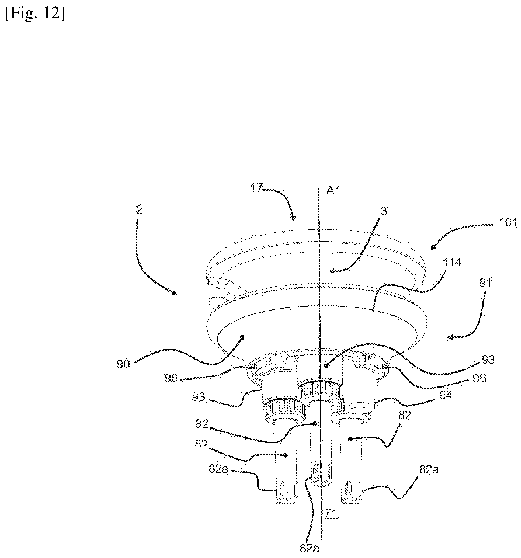

Sur les figures 11 et 12, l'enveloppe 2 comprend une collerette périphérique 90 qui est constitutive d'un couvercle d'étanchéité 91. Plus particulièrement, le couvercle d'étanchéité 91 comprend la collerette périphérique 90 qui participe de l'enveloppe 2 et une cloison d'étanchéité 92 qui est bordée par la collerette périphérique 90 dont elle constitue un fond. La cloison d'étanchéité 92 est destinée à obturer l'ouverture 75 de manière à ce que l'enceinte 71 forme une chambre d'analyse de l'eau isolée de manière étanche du reste du dispositif d'analyse 1. La cloison d'étanchéité 92 est pourvue de fûts de réception 93 des électrodes 82 dont une extrémité d'électrode 82a baigne dans l'enceinte 71. La cloison d'étanchéité 92 est aussi pourvue d'un caisson de réception 94 des moyens de stockage d'énergie électrique 16. La cloison d'étanchéité 92 est ménagée dans un plan qui est orthogonal à l'axe d'allongement A1, la collerette périphérique 90 comporte un axe de symétrie qui est confondu avec l'axe d'allongement A1. Les fûts de réception 93 et le caisson de réception 94 comportent chacun un axe de symétrie qui est parallèle à l'axe d'allongement A1. On note que le caisson de réception 94 est borgne pour garantir l'étanchéité par rapport à l'enceinte 71 tandis que les fûts de réception 93 délimitent un passage débouchant 95. Le couvercle d'étanchéité 91 comporte des pattes d'emboitement 96 qui coopèrent avec des doigts d'emboitement 76 que comprend la paroi cylindrique 73 pour permettre un assemblage de la base 70 et de la collerette périphérique 90.

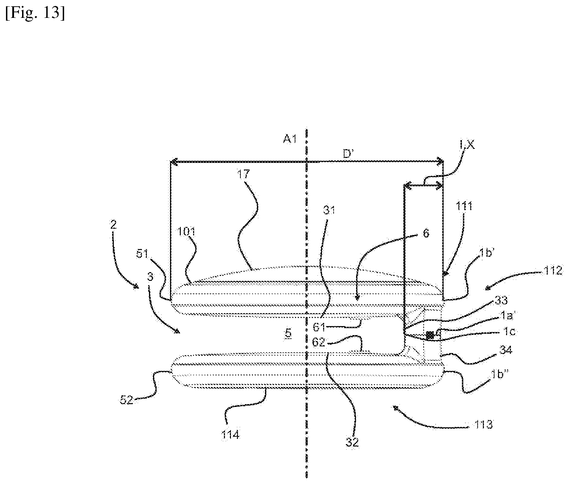

Sur la [Fig.13], l'enveloppe 2 comprend un chapeau 101 globalement conformé en U et pourvu de l'organe de préhension 3. Le chapeau 101 comprend un élément supérieur 111 formant une branche du U, un élément inférieur 112 formant l'autre branche du U et un élément intermédiaire 113 formant la base du U. L'élément supérieur 111 comporte le capot 17, la première rampe de guidage 51 qui forme une extrémité du U, la première butée de dispositif 1b', la première paroi 31 et le premier organe de détection 61. L'élément inférieur 112 comporte une embase 114 opposée axialement au capot et par l'intermédiaire de laquelle le chapeau 101 est rapporté sur le couvercle d'étanchéité 91, comme illustré sur la [Fig.12]. L'élément inférieur 112 comporte aussi la deuxième rampe de guidage 52 qui forme l'autre extrémité du U, la deuxième butée de dispositif 1b", la deuxième paroi 32 et le deuxième organe de détection 62. On note à ce stade de la description que la dimension D' est par exemple la distance prise orthogonalement à l'axe d'allongement A1 entre la première rampe de guidage 51 et la première butée de dispositif 1b', ou bien la distance prise orthogonalement à l'axe d'allongement A1 entre la deuxième rampe de guidage 52 et la deuxième butée de dispositif 1b".

L'élément intermédiaire 113 comporte la troisième paroi 33 pourvue de la butée de volet 1c et une quatrième paroi 34, radialement opposée à la troisième paroi 33, qui est pourvue du premier organe d'ancrage 1a'.

Il résulte de l'ensemble de ces dispositions que la présente invention propose avantageusement un dispositif d'analyse 1 d'un milieu liquide apte à mesurer les paramètres physico-chimiques du milieu liquide, quelle que soit la position du volet de protection 4, notamment placé en position de couverture, le dispositif d'analyse 1 étant apte à transmettre lesdits paramètres à l'unité centrale 14 pour adapter un traitement du milieu liquide si nécessaire, un tel traitement prenant notamment en compte la position du volet de protection 4 détectée par les moyens de détection 6, le dispositif d'analyse 1 étant autonome en raison de la présence du panneau photovoltaïque 15 en relation avec les moyens de stockage d'énergie électrique 16 pour alimenter en énergie électrique les moyens d'analyse 8, les moyens de contrôle 9 et les moyens de communication 10, le dispositif d'analyse 1 comprenant l'organe de préhension 3 qui est agencé en U pour délimiter l'espace 5 apte à recevoir l'extrémité 4a du volet de protection 4, l'organe de préhension étant équipé des moyens de détection 6 de la position du volet de protection 4 à l'intérieur de l'espace 5, un tel agencement en U de l'organe de préhension 3 étant tel que la distance X entre la butée de volet 1c et l'une des butées de dispositif 1b', 1b" est la plus réduite possible, notamment inférieure à 25%, voire 10% de ladite dimension D', notamment de l'ordre de quelques centimètres, de telle sorte que l'interstice I entre l'extrémité de volet 4a et le rebord R de la piscine est le plus réduit possible également, afin qu'en position de couverture, le volet de protection 4 empêche la baignade et protège le milieu liquide des rayonnements solaires, réduisant ainsi significativement un besoin de produits désinfectants dans la piscine. On comprend alors que la position du volet de protection 4 est une information importante pour le traitement automatique de l'eau de la piscine.

## Revendications

1. Dispositif d'analyse (1) d'un milieu liquide (M) comprenant une enveloppe (2) logeant des moyens d'analyse (8) du milieu liquide (M) pour mesurer des paramètres physico-chimiques du milieu liquide (M), des moyens de contrôle (9) pour gérer les paramètres mesurés, des moyens de communication (10) aptes à transmettre lesdits paramètres à une unité centrale (14), au moins un panneau photovoltaïque (15) en relation avec des moyens de stockage d'énergie électrique (16) pour alimenter en énergie électrique les moyens d'analyse (8), les moyens de contrôle (9) et les moyens de communication (10), l'enveloppe (2) comprenant un organe de préhension (3) qui est agencé en U et qui délimite un espace (5) apte à recevoir une extrémité (4a) d'un volet de protection (4), **caractérisé en ce que** l'organe de préhension (3) comprend une première paroi (31) constitutive d'une première partie étanche (11), une deuxième paroi (32) constitutive d'une deuxième partie immergée (12) comprenant une base (70) pourvue d'une pluralité de lumières (7) et logeant les moyens d'analyse (8), et une troisième paroi (33) pourvue d'une butée de volet (1c) agencée pour recevoir l'extrémité (4a) du volet de protection (4).

2. Dispositif d'analyse (1) selon la revendication 1, **caractérisé en ce que** l'organe de préhension (3) est pourvu d'un moyen de détection (6) d'une présence du volet de protection (4) à l'intérieur de l'espace (5), le moyen de détection (6) comprenant un premier organe de détection (61) équipant la première paroi (31) et un deuxième organe de détection (62) équipant la deuxième paroi (32).

3. Dispositif d'analyse (1) selon l'une quelconque des revendications précédentes, **caractérisé en ce que** la première paroi (31) borde une première chambre (41) qui loge le panneau photovoltaïque (15) et qui est surplombée par un capot (17) réalisé en une matière translucide et phosphorescente.

4. Dispositif d'analyse (1) selon l'une quelconque des revendications précédentes, **caractérisé en ce que** la deuxième paroi (32) borde une deuxième chambre (42) qui loge les moyens de contrôle (9) et les moyens de stockage d'énergie électrique (16), la deuxième chambre (42) étant partiellement délimitée par une cloison d'étanchéité (92) qui est constitutive d'un couvercle d'étanchéité (91) et qui obture une ouverture (75) de la base (70).

5. Dispositif d'analyse (1) selon la revendication 4, **caractérisé en ce que** le couvercle d'étanchéité (91) comprend une collerette périphérique (90) constitutive de l'enveloppe (2), la collerette périphérique (90) étant interposée entre un chapeau (101) du dispositif d'analyse (1) et la base (70).

6. Dispositif d'analyse (1) selon l'une quelconque des revendications précédentes, **caractérisé en ce que** la troisième paroi (32) borde une troisième chambre (43) qui loge les moyens de communication (10).

7. Dispositif d'analyse (1) selon la revendication 6, **caractérisé en ce que** la troisième chambre (43) est bordée par une quatrième paroi (34) pourvue d'un premier organe d'ancrage (1a') du dispositif d'analyse (1).

8. Ensemble (100) comprenant un dispositif d'analyse (1) selon l'une quelconque des revendications précédentes et le volet de protection (4).

9. Piscine équipée d'un ensemble (100) selon la revendication 8.

## Patentansprüche

1. Analysevorrichtung (1) für eine flüssige Umgebung (M), umfassend ein Gehäuse (2), in dem Analysemittel (8) für die flüssige Umgebung (M), um physikalisch-chemische Parameter der flüssigen Umgebung (M) zu messen, Kontrollmittel (9), um die gemessenen Parameter zu verwalten, Kommunikationsmittel (10), die die Parameter an eine Zentraleinheit (14) übertragen können, und mindestens ein Photovoltaikmodul (15) in Verbindung mit elektrischen Energiespeichermitteln (16), um die Analysemittel (8), die Kontrollmittel (9) und die Kommunikationsmittel (10) mit Strom zu versorgen, untergebracht sind, wobei das Gehäuse (2) ein Greiforgan (3) umfasst, das U-förmig angeordnet ist und einen Raum (5) abgrenzt, der ein Ende (4a) einer Schutzklappe (4) aufnehmen kann, **dadurch gekennzeichnet, dass** das Greiforgan (3) eine erste Wand (31), die aus einem ersten dichten Teil (11) besteht, eine zweite Wand (32), die aus einem zweiten eingetauchten Teil (12) besteht und eine Basis (70) umfasst, die mit einer Vielzahl von Öffnungen (7) versehen ist und in der die Analysemittel (8) untergebracht sind, und eine dritte Wand (33) umfasst, die mit einem Klappenanschlag (1c) versehen ist, der so angeordnet ist, dass er das Ende (4a) der Schutzklappe (4) aufnimmt.

2. Analysevorrichtung (1) nach Anspruch 1, **dadurch gekennzeichnet, dass** das Greiforgan (3) mit einem Erkennungsmittel (6) für das Vorhandensein der Schutzklappe (4) innerhalb des Raums (5) versehen ist, wobei das Erkennungsmittel (6) ein erstes Erkennungsorgan (61) umfasst, das die erste Wand (31) ausstattet, und ein zweites Erkennungsorgan (62), das die zweite Wand (32) ausstattet.

3. Analysevorrichtung (1) nach einem der vorhergehenden Ansprüche, **dadurch gekennzeichnet, dass** die erste Wand (31) an eine erste Kammer (41) grenzt, in der das Photovoltaikmodul (15) untergebracht ist und die von einer Abdeckung (17) aus einem lichtdurchlässigen und phosphoreszierenden Material überzogen ist.

4. Analysevorrichtung (1) nach einem der vorhergehenden Ansprüche, **dadurch gekennzeichnet, dass** die zweite Wand (32) an eine zweite Kammer (42) grenzt, in der die Kontrollmittel (9) und die elektrischen Energiespeichermittel (16) untergebracht sind, wobei die zweite Kammer (42) teilweise durch eine Dichtwand (92) begrenzt ist, die aus einem Dichtdeckel (91) besteht und eine Öffnung (75) der Basis (70) verschließt.

5. Analysevorrichtung (1) nach Anspruch 4, **dadurch gekennzeichnet, dass** der Dichtdeckel (91) einen umlaufenden Bund (90) umfasst, der das Gehäuse (2) bildet, wobei der umlaufende Bund (90) zwischen einer Kappe (101) der Analysevorrichtung (1) und der Basis (70) eingelegt ist.

6. Analysevorrichtung (1) nach einem der vorhergehenden Ansprüche, **dadurch gekennzeichnet, dass** die dritte Wand (32) an eine dritte Kammer (43) grenzt, in der die Kommunikationsmittel (10) untergebracht sind.

7. Analysevorrichtung (1) nach Anspruch 6, **dadurch gekennzeichnet, dass** die dritte Kammer (43) von einer vierten Wand (34) umgeben ist, die mit einem ersten Verankerungselement (1a') der Analysevorrichtung (1) versehen ist.

8. Baugruppe (100), umfassend eine Analysevorrichtung (1) nach einem der vorhergehenden Ansprüche und die Schutzklappe (4).

9. Schwimmbecken, das mit einer Baugruppe (100) nach Anspruch 8 ausgestattet ist.

## Claims

1. Device (1) for analysing a liquid medium (M) comprising an enclosure (2) housing means (8) for analysing the liquid medium (M) for measuring physical and chemical parameters of the liquid medium (M), control means (9) for managing the measured parameters, communication means (10) able to transmit said parameters to a central unit (14), at least one photovoltaic panel (15) connected to electrical energy storage means (16) for supplying electrical energy to the analysis means (8), the control means (9) and the communication means (10), the enclosure (2) comprising a gripping member (3) which is arranged in an U-shape and which delimits a space (5) capable of receiving an end (4a) of a protective flap (4), **characterised in that** the gripping member (3) comprises a first wall (31) constituting a first sealed part (11), a second wall (32) constituting a second immersed part (12) comprising a base (70) provided with a plurality of apertures (7) and housing the analysis means (8), and a third wall (33) provided with a flap stop (1c) arranged to receive the end (4a) of the protective flap (4).

2. Analysis device (1) according to Claim 1, **characterised in that** the gripping member (3) is provided with a means (6) for detecting the presence of the protective flap (4) inside the space (5), the detection means (6) comprising a first detection member (61) fitted to the first wall (31) and a second detection member (62) fitted to the second wall (32).

3. Analysis device (1) according to any one of the preceding claims, **characterised in that** the first wall (31) borders a first chamber (41) which houses the photovoltaic panel (15) and which is surmounted by a cover (17) made of a translucent and phosphorescent material.

4. Analysis device (1) according to any one of the preceding claims, **characterised in that** the second wall (32) borders a second chamber (42) which houses the control means (9) and the electrical energy storage means (16), the second chamber (42) being partially delimited by a sealing partition (92) which constitutes of a sealing cover (91) and which seals an opening (75) of the base (70).

5. Analysis device (1) according to Claim 4, **characterised in that** the sealing cover (91) comprises a peripheral flange (90) constituting the enclosure (2), the peripheral flange (90) being interposed between a cap (101) of the analysis device (1) and the base (70).

6. Analysis device (1) according to any one of the preceding claims, **characterised in that** the third wall (32) borders a third chamber (43) which houses the communication means (10).

7. Analysis device (1) according to Claim 6, **characterised in that** the third chamber (43) is bordered by a fourth wall (34) provided with a first anchoring member (1a') of the analysis device (1).

8. Assembly (100) comprising an analysis device (1) according to any one of the preceding claims and the protective flap (4).

9. Pool equipped with an assembly (100) according to Claim 8.
